# EUROPEAN PATENT SPECIFICATION

(11) **EP 0 669 835 B1**
(45) Date of publication and mention of the grant of the patent: **25.11.1998**
(21) Application number: 94924978.3
(22) Date of filing: 12.09.1994
(51) Int. Cl.: A61K 38/16, A23G 3/00, A23G 3/30, A23L 2/66

(54) **DIETARY HYPOCHOLESTEROLAEMIC COMPOSITIONS COMPRISING SOYA PROTEINS AND SITOSTEROLS**
DIÄTETISCHE CHOLESTERINSENKENDE ZUBEREITUNGEN, DIE SOJAPROTEINE UND SITOSTERINE ENTHALTEN
COMPOSITIONS DIETETIQUES HYPOCHOLESTEROLEMIQUES COMPRENANT DES PROTEINES DE SOJA ET DES SITOSTEROLS

(30) Priority: 21.09.1993 CH 2840/93
(43) Date of publication of application: 06.09.1995
(73) Proprietor: INPHARMA S.A., CH-6900 Lugano (CH)
(72) Inventor: FESTÖ, Norberto, CH-6900 Lugano (CH)
(74) Representative: Maspoli, René A.
(86) International application number: IB9400274
(87) International publication number: WO9508342

(56) References cited:
- GB-A- 1 427 253
- GB-A- 2 028 341

## Description

This invention refers to a hypocholesterolaemic composition for oral administration consisting of an association of soya proteins with sitosterols, both of natural origin.

The types of vascular damage that can be caused by a hypercholesterolaemic condition are numerous and a balanced diet plays a role of primary importance in the prevention of such alterations.

The populations more exposed to this type of pathology are the richer ones whose normal everyday diet, apart from being unbalanced, often exceeds daily requirements.

Hypercholesterolaemia can be treated either pharmacologically or by undertaking preventive action in terms of modifiying or integrating the usual nutritional intake.

The influence of soya proteins on nutrition is known both in normal cholesterol level and in hypercholesterolaemic conditions.

Sitosterols, beta-sitosterols in particular, are substances that are used extensively in hypocholesterolaemic traetments. Their chemical structure, similar to cholesterol, differs because of an ethyl group bound to the carbon in position 24, provides these substances with an absorption behaviour which is very different from that of cholesterol.

These substances, contrary to the cholesterol contained in food, are not absorbed and interfere with the absorption of the cholesterol taken in. The limited food absorption causes a consequent and significant hypocholesterolaemic effect.

GB-A 2 028 341, describes the use of a composition containing soya protein and pectin for reducing cholesterol level. The daily dose is 60 - 90 g/day soya protein and 1.5 - 3 g/day pectin (abstract and page 2 l.45-52).

GB-A 1 427 253, discloses an antilipemic composition comprising a nonsaponifiable fraction of soybean oil, said fraction containing about 45% by weight of plant sterols (campesterol, stigmasterol and β -sitosterol ) and about 20% by weight tocopherols (page 1 l.48-55). The use of this fraction allows to reduce dramatically the daily dose of sitosterol compared to the prior art. It is shown that 1200 - 1800 mg/day of this composition significantly lowers the cholesterol level in patients (page 1 l.69-78).

The technical problem to be solved by the invention according to this application is lowering the daily dose of sitosterol without decreasing the efficiency of the hypocholesterolaemic composition.

In GB-A 1427 253 the problem is solved by the use of a nonsaponifiable fraction of soybean oil. Although the hypocholesterolaemic effect of soya protein is disclosed in D1, there is nothing in either D1 or D2 that would suggest that soya protein and sitosterols could have a synergistic effect that would lead to a significant decrease in the daily dose of both compounds.

This invention regards the synergistic activity of the two hypocholesterolaemic substances which, if associated, can reduce the daily dose to be used in an hypocholesterolaemic diet.

The hypocholesterolaemic compositions according to the invention are characterized in the following four claims.

The ponderal ratio between soya proteins and sitosterols is not crucial and will generally be between 1 : 1 and 10 : 1.

The compositions which are the subject of the invention may also contain active principles or intergrators with coadjuvant, complementary or useful activity. Examples of these elements which can be usefully employed for the purpose are: mineral salts, vitamins, digestive enzymes, soluble fibres, insoluble fibres, amino acids, phospholipids.

The compositions which are the subject of the invention are prepared using traditional techniques and excipients. These compositions are prepared by mixing soya proteins and sitosterols with excipients that are physiologically suitable and with pleasant appearance, colour and flavour. Among the excipients available for alimentary use are diluting agents, sweeteners, binding agents, flavouring agents, lubricants, antiaggregating agents, natural and synthetic colouring agents, solubilizers, surfactants and preservatives.

Examples of diluting substances are: microcrystalline cellulose, lactose, glycine, rice starch, saccharose, fructose, sorbitol; examples of binding agents are: starch water, pvp, methylcellulose; examples of flavouring agents are: citric acid, tartaric acid, sodium chloride, sodium glutamate, menthol ; examples of lubricants are: PEG, magnesium stearate, stearic acid, talc.

The examples of dietary compositions include: chewable, effervescent, swallowable and coated tablets; film coated pills, capsules, soft gelatine capsules, syrup, fruit beverages, granule sachets, fruit gelatines, sweets.

The following are hypocholesterolaemic dietary formulations:

| Coated tablets | |
|---|---|
| Nucleus: | |
| Soya proteins | 500 mg |
| sitosterols | 100 mg |
| lactose | 90 mg |
| magnesium stearate | 10 mg |

| Coating: | |
|---|---|
| Methylcellulose | 10 mg |
| titanium dioxide | 2 mg |
| PEG 6000 | 3 mg |
| talc | 5 mg |

| Tablets | |
|---|---|
| Soya proteins | 300 mg |
| sitosterols | 200 mg |
| maize starch | 30 mg |
| pvp | 20 mg |
| lactose | 50 mg |
| talc | 10 mg |
| magnesium stearate | 5 mg |

| Granules for extemporaneous suspension | |
|---|---|
| Soya proteins | 800 mg |
| sitosterols | 200 mg |
| saccharose | 2000 mg |
| sodium saccharinate | 20 mg |
| apple flavouring | 50 mg |

| Capsules | |
|---|---|
| Soya proteins | 100 mg |
| sitosterols | 100 mg |
| magnesium stearate | 10 mg |
| silica precipitate | 5 mg |

| Shell: | |
|---|---|
| gelatin | 100 mg |

| Chewable tablets | |
|---|---|
| Soya proteins | 300 mg |
| sitosterols | 200 mg |
| mannitol | 500 mg |
| pvp | 20 mg |
| sorbitol | 100 mg |
| aspartame | 3 mg |
| magnesium stearate | 5 mg |

The dietary preparations based on soya proteins/sitosterols can be associated with substances such as food fibres (soluble of insoluble) to produce dietary hypocholesterolaemic-bulking agents.

The following is an example of a formulation:
Hypocholesterolaemic-bulking agent

| Granules for extemporaneous suspension | |
|---|---|
| Soya proteins | 800 mg |
| sitosterols | 200 mg |
| guar gum | 2000 mg |
| saccharose | 1000 mg |
| sodium saccharinate | 20 mg |
| orange flavouring | 50 mg |

The hypocholesterolaemic association combined with enzymes of vegetable origin such as bromelain gives hypocholesterolaemic-digestive products.

The following is an example of a formulation
Hypocholesterolaemic-digestive product

| Chewable tablets | |
|---|---|
| Soya proteins | 300 mg |
| sitosterols | 200 mg |
| bromelain | 10 mg |
| mannitol | 500 mg |
| pvp | 20 mg |
| sorbitol | 100 mg |
| aspartame | 3 mg |
| magnesium stearate | 5 mg |

## Claims

1. Hypocholesterolaemic compositions with soya proteins associated synergistically with sitosterol , characterized in that said compositions contain from 50 mg to 2000 mg of Soya proteins and from 10 mg to 1000 mg of sitosterols per unit, both of natual origin.

2. Compositions according to Claim 1 , containing other active principles or integrators with a coadjuvant, complementary or useful activity.

3. Compositions according to Claims 1 or 2, containing also at least one substance chosen from the group comprising: mineral salts, vitamins, digestive enzymes, soluble fibres, insoluble fibres, amino acids and phospholipids.

4. Compositions according to any of the previous Claims in the form of chewable, effervescent, swallowable and coated tablets, capsules, soft gelatine capsules, syrup, fruit beverages, granule sachets, fruit gelatines, sweets.

## Patentansprüche

1. Hypocholesterinämische Zusammensetzungen mit Sojaproteinen, die auf synergistische Weise mit Sitosterin assoziiert sind, dadurch gekennzeichnet, daß die Zusammensetzungen 50 mg bis 2000 mg Sojaproteine und 10 bis 1000 mg Sitosterine pro Einheit enthalten, die beide natürlichen Ursprungs sind.

2. Zusammensetzungen gemäß Anspruch 1, die andere aktive Prinzipien oder Integratoren mit einer Hilfsmittelaktivität, einer komplementären oder brauchbaren Aktivität enthalten.

3. Zusammensetzungen gemäß den Ansprüchen 1 oder 2, die wenigstens eine Substanz enthalten, die aus der Mineralsalze, Vitamine, verdauungsfördernde Enzyme, lösliche Fasern, unlösliche Fasern, Aminosäuren und Phospholipide umfassenden Gruppe ausgewählt ist.

4. Zusammensetzungen gemäß irgendeinem der vorhergehenden Ansprüche in Form von Kau-, Brause-, Schlucktabletten und Dragees, Kapseln, Weichgelatine-Kapseln, Sirup, Fruchtgetränken, Granulatkissen, Fruchtgelatinen und Bonbons.

## Revendications

1. Compositions hypocholestérolémiques contenant des protéines de soja associées de manière synergique avec des sitostérols, caractérisées en ce que lesdites compositions contiennent de 50 mg à 2 000 mg de protéines de soja et de 10 mg à 1 000 mg de sitostérols par unité, tous d'origine naturelle.

2. Compositions selon la revendication 1, contenant d'autres principes actifs ou intégrateurs à activité de coadjuvant, complémentaire ou utile.

3. Compositions selon la revendication 1 ou 2, contenant aussi au moins une substance choisie dans le groupe comprenant : les sels minéraux, les vitamines, les enzymes digestives, les fibres solubles, les fibres insolubles, les acides aminés et les phospholipides.

4. Compositions selon l'une quelconque des revendications précédentes sous forme de comprimés pouvant être mâchés, effervescents, pouvant gonfler et enrobés, de capsules, de capsules de gélatine molle, de sirop, de boissons à base de fruits, de sachets de granulés, de gélatines de fruits, de bonbons.
